(19) 

Europäisches
Patentamt
European
Patent Office
Office européen
des brevets

(11) **EP 4 679 452 A1**

(12) **EUROPEAN PATENT APPLICATION**

(43) Date of publication:
**14.01.2026 Bulletin 2026/03**

(21) Application number: **25183406.5**

(22) Date of filing: **17.06.2025**

(51) International Patent Classification (IPC):
**G16H 50/20** (2018.01)      **G16H 50/30** (2018.01)

(52) Cooperative Patent Classification (CPC):
**G16H 50/20; G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **11.07.2024 US 202418769596**

(71) Applicant: **Optellum Limited**
**Oxford, Oxfordshire OX1 4EH (GB)**

(72) Inventors:
• **ARTETA MONTILVA, Carlos Federico**
  **Oxford, OX1 4EH (GB)**
• **WATERFIELD PRICE, Noah**
  **Oxford, OX1 4EH (GB)**
• **GASIMOVA, Aydan**
  **Oxford, OX1 4EH (GB)**

(74) Representative: **Optimus Patents Limited**
**Peak Hill House**
**Steventon**
**Basingstoke, Hampshire RG25 3AZ (GB)**

(54) **SYSTEM AND METHOD TO AID CLINICIANS IN ASSESSING THE OUTCOME OF LUNG CANCER INTERVENTIONS**

(57)     A Computer Aided Diagnosis, CADx, system and method for predicting an outcome score for a patient is described. The system comprising: an input circuit configured to receive input data comprising at least one input medical image for a patient; an outcome prediction circuit operably coupled to the input circuit configured to receive input data from the input circuit for outcome prediction analysis; wherein the outcome prediction circuit is further configured to receive details of a suggested future intervention for the patient; and the input data to the outcome prediction circuit is analysed to generate the outcome score for the patient accounting for the details of the future intervention.

FIG. 1

EP 4 679 452 A1

## Description

## Field of Invention

This invention relates to the field of Computer Aided Diagnosis (CADx) devices and methods for assisting the interpretation of medical images to support clinicians in healthcare.

## Background of Invention

In the field of medical imaging, a variety of technologies can be used to investigate biological processes and anatomy. The following examples are types of scan that may be used to provide medical images: X-Ray; Computed Tomography (CT); Ultrasound (US); Magnetic Resonance Imaging (MRI); Single Photon Emission Tomography (SPECT); and Positron Emission Tomography (PET). Each type of scan is referred to as an "imaging modality".

Typically, a scan provides a "dataset". The dataset comprises digital information about the value of a variable at each of a plurality of spatial locations in either a two-dimensional or (more typically) a three-dimensional space. As a specific example, a CT scan may provide images of the chest of a patient. Such a CT scan might, as a more specific example, show lung nodules in the chest.

Computer Aided Detection (CADe) devices serve to assist clinicians in assessing the medical images. CADe devices need to provide a clinician with standardised, objective and repeatable information. The information typically relates to particular anatomical regions, including both normal tissue and lesions, within a person. CADe devices may be used as a so-called 'Second Reader' device. Second Reader Devices are based on an approach whereby a radiologist first looks at an image resulting from a scan, for example a mammogram. The radiologist will then, based on training and experience, identify areas of the scan where the radiologist considers that there may need to be a further investigation, for example a biopsy. However, the radiologist can then consider the CADe findings. Those findings might involve a display to highlight any additional suspicious regions on the mammogram. The radiologist will then, based on training and experience, look at those further areas of the scan. The CADe device is thereby performing a second look at the scan. The results of the second look at the scan may be that the radiologist will be directed to areas of the scan that he/she had overlooked. In this way, CADe devices are designed to reduce 'false negatives', which are also termed 'missed findings'. Thus, CADe devices perform a support role to clinicians. Examples of commercial CADe devices for lung health include 'Veolity Lung™' from MeVis, 'Veye Chest™' from Aidence, and 'Lung AI™' from Arterys.

Computer Aided Diagnosis (CADx) devices are a related technology to CADe. CADx devices attempt to solve a different problem and relate generally to risk assessment. Instead of focussing on potentially missed findings as in CADe, they try to assist the user to classify findings correctly, for instance, either as malignant or benign in the case of potentially cancerous lesions. They rely on the user to identify abnormalities, and typically provide a score that is indicative of the risk of malignancy. Examples of commercial CADx devices are the breast cancer screening product 'Transpara™' from 'Screenpoint™', and the lung cancer product Virtual Nodule Clinic (VNC) from Optellum. There are many non-clinical CADx devices in the academic literature.

In the space of lung cancer, CADx devices have traditionally focused on evaluating lung nodules to produce malignancy risk scores, with the aim of helping clinicians make more appropriate decisions for the patient. Examples of such lung cancer CADx devices include the VNC from Optellum, and qCT-Lung from qure.ai. The connection between risk assessment and clinical decision-making lies in that the most appropriate next step for a given patient with a lung nodule depends on the risk of it being malignant. For example, higher risk cases would warrant shorter term actions (e.g., PET/CT, biopsy, or short-term CT follow-up), while lower risk cases can be addressed with longer term CT follow-ups. The consensus of experts on how to link risk and clinical decision is collected in clinical guidelines published regularly by the relevant medical societies. In the case of lung cancer, these guidelines include those from the Fleischner Society [1]. In these guidelines, risk assessment of the lung nodule is central to the decision-making process, hence it is thought that CADx devices that help clinicians better assess risk can lead to better management.

The first stage of the management process is *disease identification*, where the primary aim is to ascertain whether lung cancer is present and to decide what further diagnostic procedures (if any) are needed, i.e. when the initial CT (and any other pre-biopsy results) is being reviewed. The aim of the assessment is to work out whether cancer is considered likely present, absent, or uncertain, and the decision is whether and how to investigate further. Disease identification may occur multiple times, as it will likely be repeated each time new information is gained. For instance, if a clinician decided to follow up a patient after six months, they would then have two CTs taken six months apart to consider and could use this new information to again perform disease identification. The result of disease identification in the context of cancer is a positive or negative identification of malignancy with sufficient certainty to either discharge the patient or proceed to disease characterisation. This result is referred to as a malignancy diagnosis.

The second decision point is *disease characterisation*, at which point lung cancer is known to be present or strongly suspected to be present. Here, the primary aim is to determine the characteristics of the cancer, and the decision is how to manage and/or treat the disease appropriately, i.e. once the biopsy results are available and have confirmed the existence of cancer, the characteristics of cancer, such as the type of lung cancer or how advanced it is, must be determined to decide how it can be best managed and/or treated.

An extension of disease characterisation, which also considers a proposed treatment, is *outcome prediction*. The aim of outcome prediction is to estimate the prognosis of a cancer after treatment and/or predict the response of the cancer to treatment. For instance, assessing the risk of the cancer recurring after treatment is an important factor when planning the treatment of cancer.

Characterising the lung cancer risk of recurrence plays an important role in decision making in both the pre- and post-treatment setting. Recurrence can occur locally, regionally, or in distant locations. Local recurrence refers to the presence of a tumour within the initial primary organ; regional recurrence occurs in the adjacent organ or lymph nodes draining the organ, encompassing ipsilateral lymph nodes (including supraclavicular lymph nodes), the bronchial stump, and the pleura and chest wall [2]; distant recurrence occurs in locations beyond regional. The most frequently observed sites for distant metastases in recurrent lung cancer are similar to those identified during the initial diagnosis, namely the brain, bone, liver, and adrenal glands [3].

Early-stage non-small-cell lung cancer (NSCLC) is primarily treated with surgical resection of the primary tumour. However, the rate of lung cancer recurrence following curative surgical resection is 30-55% [4-6] and remains a significant challenge in patient management. Accurate prediction of recurrence risk of early-stage lung cancer from clinical and pathological patient data can help clinicians to make informed decisions regarding patient treatment and follow-up. For instance, recurrence prediction can guide treatment decisions and follow-up strategies, such as the use of neoadjuvant or adjuvant chemotherapy and/or immunotherapy, the use of stereotactic radiation body therapy (SBRT) or surgery for primary treatment, the extent of lung resection for surgical candidates, and the timing of follow-up blood tests and CTs.

The utility of different disease characterisations or outcome predictions depends on where in the diagnostic/treatment pathway the patient is, whether it is pre-diagnosis, post-diagnosis but pre-treatment, or post-treatment. The relevant disease characterisations and outcome predictions will also vary dependent on the type of treatment, for instance, surgical resection versus chemoradiotherapy.

Characteristics that are useful in characterising lung cancer treatment response include but are not limited to:

Histological subtype: Lung cancer can be classified into many histological subtypes based upon its microscopic appearance. These histological subtypes can differ significantly in their pathogenesis. For instance, small-cell lung cancer tends to grow and spread faster than non-small-cell lung cancer, has a high recurrence rate, but responds well to treatment. The two most common subtypes of non-small-cell lung cancer - adenocarcinoma and squamous-cell carcinoma - differ in prognosis, in recurrence rate, and in what drugs are used to treat them.

Expression of certain proteins: Certain cancers express the PD-L1 protein to a greater extent than others and are associated with high tumour aggressiveness and a worse prognosis but are also more responsive to certain treatments.

The presence of certain mutations: There are also targeted therapies that are effective in cases where certain genetic mutations can be identified, such as epidermal growth factor receptor (EGFR)

Clinical cancer stage: The extent and advancement of lung cancer is classified according to its TNM stage, which represents the size and extent of the main tumour (T), the number of nearby lymph nodes the cancer has spread to (N), and whether the cancer has metastasised to other parts of the body (M). There is also an overall stage which combines all three components of the TNM stage.

Tumour Location: The location of a tumour within the lung can influence the risk of recurrence. Peripheral tumours may be associated with a different prognosis compared to those located centrally (and closer to pulmonary lymph nodes) or near vital structures. The accessibility of the tumour for surgical resection and the likelihood of complete removal without residual disease also play a role in determining the risk of recurrence.

These cancer characteristics are equally useful in characterising post-treatment risk of recurrence, in addition to the following post-treatment cancer characteristics:

Extent of residual disease post-surgery: The R-classification of residual disease post-surgery has been shown to have prognostic relevance and is defined as the completeness of resection. R0- no residual tumour, R1- residual microscopic tumour, R2- residual macroscopic tumour [7]

Lung tumour and surrounding tissue microbiome and transcriptome: 40-60% of patients will not respond, or will develop resistance to, immunotherapy [8]. Transcriptionally active microbiota within the bronchoalveolar lavage fluid (BALF) are being investigated for their association with anti-tumour immunity and response to PD-1 blockade [9].

Minimal Residual Disease (MRD): The presence of MRD after surgical resection can be a critical indicator of the risk of lung cancer recurrence. MRD is the small number of cancer cells that remain in the body after treatment and may not be detectable with standard imaging tests. Techniques such as liquid biopsy and molecular assays are used to identify MRD.

These considerations are becoming increasingly important with the rise of personalised medicine, which aims to base clinical decisions about the treatment and management of cancer based on its cancer characteristics.

Many of the cancer characteristics are often only measurable by performing tests directly on cancer tissue. This does not necessarily require any further tissue extraction procedures, as the tissue from the earlier disease identification biopsy can sometimes be used for disease characterisation. However, if an insufficient amount of tissue was extracted to perform all the necessary disease characterisation tests, then another procedure, such as a second tissue biopsy, may be required.

Using CT images to inform disease characterisation could both supplement the results of other disease characterisation procedures and partly address the lack of biopsy results when they are not available and/or not possible to obtain. This could also help alleviate some of the issues with invasive, tissue-based tests. Firstly, lung CT imaging is a non-invasive, low-risk, and cost-effective procedure that is already routinely used in clinical management of patients with pulmonary nodules. These images could be reused for image-based disease characterisation and thus obviate the need for further diagnostic procedures. Secondly, a CT-based approach would be robust to tumour heterogeneity as a CT image provides a comprehensive view of the tumour as it captures the entire lesion and its surrounding environment. Thirdly, it could be applied to all nodules visible on a CT therefore would not have the same late-stage limitation that liquid biopsy has and could be applied to nodules that cannot be tissue biopsied because they are too small or inaccessible. Finally, the results could be obtained almost immediately, taking only the amount of time required to process the image, rather than days or weeks associated with pathological analysis.

Machine learning models such as neural networks represent a good alternative for predicting risk of recurrence from medical images to manual, qualitative assessment of single, human-defined parameters. These models can learn, directly from data, what patterns in the nodule and surrounding lung image to consider such that the discrimination between negative recurrence and positive recurrence is maximised. Automatic assessment of medical images in this way can account for these complex but meaningful patterns in the data that are difficult to express and quantify by humans. For instance, the appearance of any nodule spiculations, which are long tendril-like structures extending away from the nodule edge, could be informative for predicting histology but is difficult to quantify in a manner that is reproducible across medical practitioners. Another example is the appearance of intrapulmonary, mediastinal and extrathoracic lymph nodes, where the presence of malignant lymph nodes is informative for staging and prognosis but are not always consistently biopsied due to the expertise required.

[1] MacMahon, Heber, et al. "Guidelines for management of incidental pulmonary nodules detected on CT images: from the Fleischner Society 2017." Radiology 284.1 (2017): 228-243.

[2] Yano T, Yokoyama H, Inoue T, et al. The first site of recurrence after complete resection in non-small-cell carcinoma of the lung. Comparison between pN0 disease and pN2 disease. J Thorac Cardiovasc Surg. 1994;108(4):680-3.

[3] Cangemi V, Volpino P, D'Andrea N, et al. Local and/or distant recurrences in T1-2/N0-1 non-small cell lung cancer. Eur J Cardiothorac Surg. 1995;9(9):473-8.

[4] Sugimura H et al. Survival after recurrent NSCLC after complete pulmonary resection. Annals of Thoracic Surgery, 2007.

[5] Hoffman PC, Mauer AM et Vokes EE. Lung cancer. Lancet, 2000.

[6] Carnio S et al. Prognostic and predictive biomarkers in early stage non small-cell lung cancer: tumor based approaches including gene signatures. Translational Lung Cancer Research, 2013.

[7] D.J. Giroux, R. Rami-Porta, K. Chansky, et al. The IASLC Lung Cancer Staging Project: data elements for the prospective project, J Thorac Oncol, 4 (2009), pp. 679-683

[8] Gandhi L, Rodriguez-Abreu D, Gadgeel S, Esteban E, Felip E, De Angelis F, et al. Pembrolizumab plus Chemotherapy in Metastatic Non-Small-Cell Lung Cancer. N Engl J Med 2018;378(22):2078-92 doi 10.1056/NEJMoa1801005.

[9] Tsay JJ, Wu BG, Sulaiman I, Gershner K, Schluger R, Li Y, Yie TA, Meyn P, Olsen E, Perez L, Franca B, Carpenito J, Iizumi T, El-Ashmawy M, Badri M, Morton JT, Shen N, He L, Michaud G, Rafeq S, Bessich JL, Smith RL, Sauthoff H, Felner K, Pillai R, Zavitsanou AM, Koralov SB, Mezzano V, Loomis CA, Moreira AL, Moore W, Tsirigos A, Heguy A, Rom WN, Sterman DH, Pass HI, Clemente JC, Li H, Bonneau R, Wong KK, Papagiannakopoulos T, Segal LN. Lower Airway Dysbiosis Affects Lung Cancer Progression. Cancer Discov. 2021 Feb;11(2):293-307. doi: 10.1158/2159-8290.CD-20-0263. Epub 2020 Nov 11. PMID: 33177060; PMCID: PMC7858243.

**Summary of the invention**

According to an embodiment there is provided a Computer Aided Diagnosis, CADx, system for predicting an outcome score for a patient, comprising: an input circuit configured to receive input data comprising at least one input medical image for a patient; an outcome prediction circuit operably coupled to the input circuit configured to receive input data from the input circuit for outcome prediction analysis; wherein the outcome prediction circuit is further configured to receive details of a suggested future intervention for the patient; and the input data to the outcome prediction circuit is analysed to generate the outcome score for the patient accounting for the details of the future intervention.

Preferably, the output prediction circuit comprises a disease characterisation model and an outcome prediction model; and the outcome prediction circuit is configured to receive input data from the input circuit for analysis by the disease characterisation model; wherein output from the disease characterisation model is provided to the outcome prediction model, to generate the outcome score for the patient.

In a preferred embodiment, the outcome prediction model is further configured to receive at least one or more historical intervention records for the patient.

Further preferably, at least one of the historical intervention records and the future intervention are set as a null value, and are considered as no-interventions towards calculations of the outcome score.

In an embodiment, the outcome score comprises at least one of score of disease recurrence and treatment response.

Preferably, the outcome score further comprises one or more different characterizations of the outcome.

Further preferably, the one or more different characterisations of the outcome comprise one or more of: a malignancy score, a disease recurrence score, a disease recurrence location, a disease recurrence time, and an adverse effects score.

Preferably, the outcome score is provided for a predefined time sequence between 1 month and 10 years.

In a preferred embodiment. the input medical image is at least one of a CT scan, X-ray scan, ultrasound scan, MRI scan, SPECT scan, PET scan, in which all or part of a patient's lungs are visible.

Further preferably, the outcome prediction circuit further comprises an intervention encoder, and the at least one intervention record and the details of the future intervention are provided to the intervention encoder, which encodes the intervention record and details of the future intervention before providing it to the outcome prediction model.

Preferably, the intervention encoder is trained to map raw intervention information into a vector to enhance semantics of the raw intervention information.

Further preferably, the intervention record is provided to the intervention encoder in the form of an intervention type vector and an intervention characteristic vector.

In a preferred embodiment, the mapping of the raw intervention information into a vector is an identity mapping that does not change the intervention type vector or the intervention characteristic vector.

Preferably, the disease characterization model comprises at least one of: an image characterization model for analysing visual features of an input image; and a structured data characterization model to encode structured data into a mathematical representation.

Further preferably the disease characterization model further comprises a fusion model to aggregate an output from the image characterization model and the structured data characterization model.

In a preferred embodiment, one or more of the disease characterisation models and the outcome prediction model are trained using machine learning.

Further preferably, the input data is provided as a longitudinal data sequence with each input in the sequence having an associated timestamp.

Preferably, a disease characterization model will provide an output for each input in the longitudinal data sequence, and the fusion module will provide a single aggregated output.

In a preferred embodiment, the intervention record is a record of all interventions performed for the patient, along with a timestamp for each intervention; where the interventions in the intervention record and the future intervention comprise at least one of: surgery, radiotherapy, chemotherapy, immunotherapy, ablation, laser therapy, cryotherapy, diathermy, and photodynamic therapy, and no intervention.

According to an embodiment there is also provided a computer implemented method for predicting an outcome score for a patient, using a computer Aided Diagnosis, CADx, system comprising the steps of: receiving, at an input circuit, input data comprising at least one input medical image for a patient; receiving at an outcome prediction circuit operably coupled to the input circuit, input data from the input circuit, wherein the input data is analysed by the outcome prediction circuit ;wherein the outcome prediction circuit is configured to receive details of a future intervention for the patient; and analysing the inputs to the outcome prediction circuit to generate an outcome score for the patient accounting for the details of the future intervention.

## Brief Description of the Figures

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

Figure 1: General configuration of the CADx system for outcome prediction;

Figure 2: General configuration of the CADx system for outcome prediction with outcome characterization;

Figure 3a: Example configuration of the disease characterization model;

Figure 3b: Example configuration of the disease characterization model for longitudinal inputs;

Figure 4a: Example configuration of the outcome prediction model with outcome characterization;

Figure 4b: Example configuration of the outcome prediction model with outcome characterization;

Figure 5a: Example configuration of the intervention encoder;

Figure 5b: Example configuration of the intervention encoder with intervention record;

Figure 6a: Embodiment of the CADx system for pre-diagnostic lung cancer recurrence prediction;

Figure 6b: Embodiment of the CADx system for pre-surgical lung cancer recurrence prediction;

Figure 6c: Embodiment of the CADx system for post-operative lung cancer recurrence prediction;

Figure 6d: Embodiment of the CADx system for lung cancer pre-treatment response prediction;

Figure 7a: Example training diagram of the CADx system for outcome prediction based on machine learning models;

Figure 7b: Example training diagram of the CADx system for outcome prediction and outcome characterization based on machine learning models;

## Detailed Description

## Overview of the invention

This invention describes a CADx system (100) for lung cancer outcome prediction, and its objective is to assist clinicians in the planning of lung cancer treatment. The CADx system (100) analyses medical images of a patient, optionally together with biomarkers, patient parameters, intervention history and images from other modalities, such as microscopy (e.g. histology), to predict an outcome score indicative of the likelihood that a particular outcome of the lung cancer will occur given a new clinical intervention (e.g. disease treatment). For example, in an embodiment of this invention, such an outcome is the recurrence of the lung cancer following a clinical intervention. In another embodiment of the invention, the outcome is treatment response following a clinical intervention. For instance, outcome prediction can be the pathologic response following neo-adjuvant immunotherapy and/or chemotherapy, or a prediction of adverse effects given a specific treatment plan.

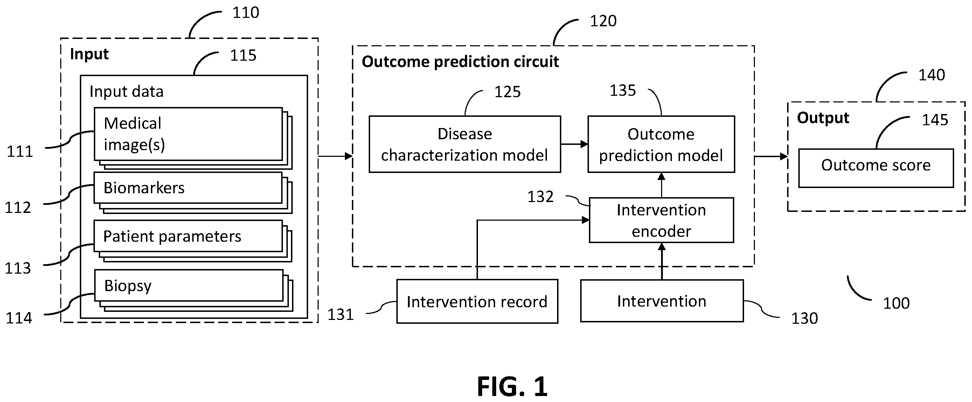

The CADx system (100), as shown in FIG 1, includes: an input circuit (110) which parses input data (115) such that it can be processed by an outcome prediction circuit (120); an outcome prediction circuit (120) containing a disease characterization model (125) that processes the input data provided by the input circuit (115), and an outcome prediction model (135) which predicts an outcome score (145) given an intervention (130) encoded by an intervention encoder (132) and a disease characterization (125); and an output circuit (140), which takes the outcome score (145) output of outcome prediction circuit (120) and presents it to the user. In an embodiment, there is provided a Computer Aided Diagnosis, CADx, system (100) for predicting an outcome score for a patient, comprising: an input circuit (110) configured to receive input data comprising at least one input medical image for a patient; an outcome prediction circuit (120) operably coupled to the input circuit (110) configured to receive input data from the input circuit for outcome prediction analysis; wherein the outcome prediction circuit (120) is further configured to receive details of a suggested future intervention (130) for the patient; and the inputs to the outcome prediction circuit are analysed to generate an outcome score (145) for the patient accounting for the details of the future intervention. In another embodiment, there is provided a computer implemented method for predicting an outcome probability for a patient, using a computer Aided Diagnosis, CADx, system comprising the steps of: receiving, at an input circuit, input data comprising at least one input medical image for a patient; receiving at an outcome prediction circuit operably coupled to the input circuit, input data from the input circuit, wherein the input data is analysed by the outcome prediction circuit ; wherein the outcome prediction circuit is configured to receive details of a future intervention for the patient; analysing the inputs to the outcome prediction circuit to generate an outcome score for the patient accounting for the details of the future intervention

The input data in the input circuit (115) typically consist of at least one medical image of a patient showing their lungs. Preferably the input medical image is at least one of a CT scan, X-ray scan, ultrasound scan, MRI scan, SPECT scan, PET scan, in which all or part of a patient's lungs are visible. Biomarkers from liquid and/or tissue assays (112), patient clinical parameters (113) such as age and sex as collected in hospital systems into a preferred format for analysis, and results of biopsy (114) performed on the lung lesions being assessed.

In some embodiments of the invention, the outcome prediction circuit also accepts an intervention record (131) containing information of previous lung cancer interventions, and which is parsed by the outcome prediction model (135) as encoded by the intervention encoder (132) to compute an outcome score (145) given a next intervention (130). Preferably, the outcome prediction model (130) is further configured to receive at least one or more historical intervention records (131) for the patient.

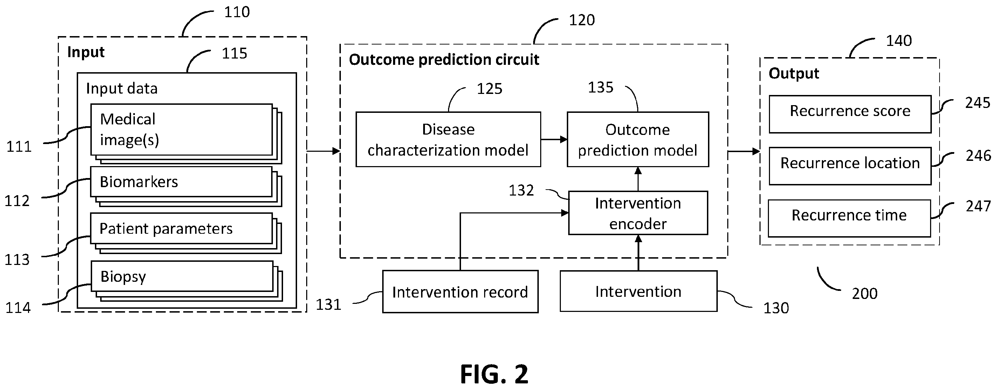

In further embodiments of this invention, the outcome prediction circuit (120) estimates additional outcomes related to the main outcome score. Preferably, the outcome score comprises at least one of probability of disease recurrence and treatment response. For example, when the outcome score (145) is a lung cancer recurrence score (245), an additional outcome is an indication of the location of the recurrence (246) (e.g. local, regional, or distant), or an estimate of the time for recurrence (247), as seen in FIG 2 (200). In an embodiment, the outcome score further comprises one or more different characterizations of the outcome. Preferably, the one or more different characterisations of the outcome comprise one or more of: a malignancy score, a disease recurrence score, a disease recurrence location, a disease recurrence time, and an adverse effects score.

The present invention fits various existing clinical workflows such as a pre-diagnostic lung cancer outcome prediction, where a lung cancer diagnostic has not been confirmed but the score can inform the diagnostic method; a pre-treatment scenario, where the lung cancer diagnosis is confirmed but has not been treated; and a post-treatment scenario where the lung cancer has been treated with at least one of surgical resection, radiotherapy, chemotherapy, targeted therapy, immunotherapy, ablation, laser therapy, cryotherapy, diathermy, or photodynamic therapy. In real-world cancer care, patients often undergo multiple rounds of therapy, each informed by the outcome of the previous round, for instance, a patient may undergo concurrent chemotherapy and radiotherapy, followed by immunotherapy. Then, the cancer may recur in another lung lobe, which may then be treated (or "salvaged") with surgery, followed by consolidation radiotherapy of the recurrence. With this level of heterogeneity in lung cancer treatment pathways, it is critical that an outcome prediction device can adapt to a broad range of interventions and outcomes.

Being able to utilize a CADx system for outcome prediction across such a range of clinical workflows for lung cancer treatment requires the flexibility offered by the outcome prediction circuit (120) of this invention. For example, the flexibility to condition the outcome score on a next intervention, as done with the combination of the outcome prediction model (135) and the intervention encoder (132), or furthermore, accounting for the history of interventions as captured by the intervention record (131).

The various elements of the CADx system (100) for lung cancer outcome prediction are detailed next.

### Outcome prediction circuit

The outcome prediction circuit (120) is the central element of the CADx system (100) for lung cancer outcome prediction. Its functionality is to take a set of input data (115), containing at least a medical image showing a lung cancer lesion, or potential lung cancer lesion, and compute a score indicating the likelihood of a specific outcome would occur following treatment with a given clinical intervention (130).

In its general form, the outcome prediction circuit (120) implements methodology related to two key functionalities: disease characterization (125) and outcome prediction (135) for a given intervention (130).

Preferably the output prediction circuit (120) comprises a disease characterisation model (125) and an outcome prediction model (135); and the outcome prediction circuit is configured to receive input data from the input circuit for analysis by the disease characterisation model; wherein the output from the disease characterisation model is provided to the outcome prediction model, to generate the outcome score for the patient.

### Disease characterization

Disease characterization is performed by a disease characterization model (125), which captures the statistical patterns in the input data (115) and embeds them into a mathematical representation. In some examples of this invention, the patterns in the input data (115) are represented as d-dimensional vectors $v$. The output representation of the disease characterization model (125), $v$, can be interpreted as a summary of the contents of the input data. For example, providing a rich description of the visual appearance of the suspicious lesion or diagnosed cancer in the medical image, its progression over time if serial imaging is available, and any relevant information from other modalities present in the input data that may be relevant for the downstream task of predicting an outcome of the lung cancer given a treatment. Such characteristics include lesion characteristics such as size, spiculation, radiological attenuation, location (e.g. relative to anatomical structures), temporal progression, textural patterns, and contextual characteristics such as the appearance of the lung tissue in the surroundings of the lesion, appearance of the lymph nodes, and description of other lesions present in the patient.

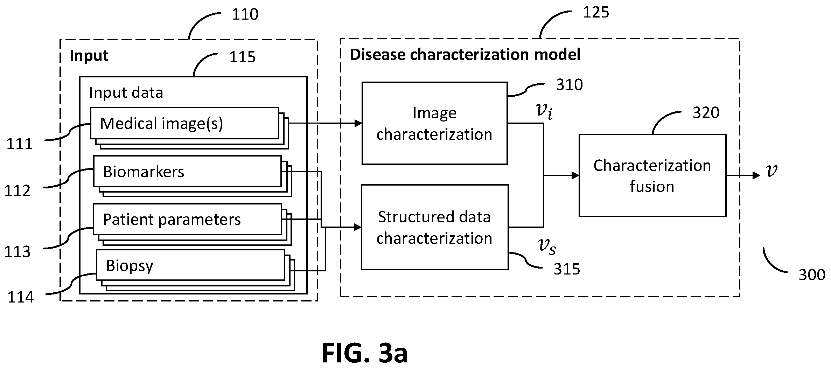

In some examples of this invention, the disease characterization model is a composition of models targeting different input data modalities. For example, in FIG 3a, a first disease characterization model (310) is dedicated to raw medical images, producing a characterization $v_i$ that summarizes visual patterns in the input images, while a second characterization model (315) is dedicated to structured data, producing a characterization $v_s$ that encodes the structured data into a mathematical representation. Finally, a third model for characterization fusion (320) aggregates $v_i$ and $v_s$ to produce a final characterization $v$ of the entire input data (115). In an embodiment, the disease characterization model comprises at least one of: an image characterization model for analysing visual features of an input image; and a structured data characterization model to encode structured data into a mathematical representation. Preferably, the disease characterization model further comprises a fusion model to aggregate the output from the image characterization model and the structured data characterization model.

In some examples of the disease characterization model, the image characterization (310) is performed by a machine learning model such as a neural network. In some examples, this neural network (310) is trained as an image auto-encoder, while in other examples it is trained end-to-end from the error signal produced by predicting an outcome score (145) on retrospectively collected training data. In another example, the structured data characterization model (315) is an encoder that transforms structured data into a vectorial form, for example, by one-hot encoding categorical data, and quantizing and encoding continuous variables. In another example, the structured data characterization is further composed by a neural network that transforms an initial encoding of the structured data into a second encoding of the structured data based on the error signal produced by predicting an outcome (145) on retrospectively collected training data. In another example, the characterization fusion (320) aggregation function consists of a concatenation of $v_i$ and $v_s$, while in a different example the characterization fusion is performed by a dedicated neural network that takes $v_i$ and $v_s$ as inputs, and outputs $v$ based on an optimization process guided by the error signal produced by predicting an outcome (145) on retrospectively collected training data.

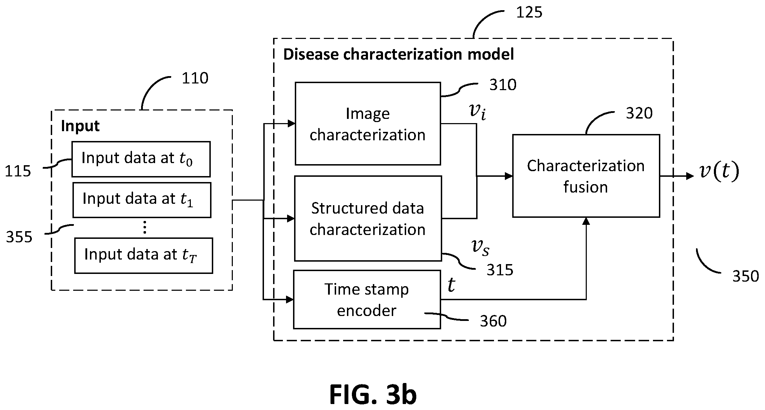

In some examples of this invention (350) depicted in FIG 3b, the disease characterization model accepts longitudinal data, that is, a sequence (355) of input data (115) points with an associated time stamp of when they were acquired. In an embodiment, the input data is provided as a longitudinal data sequence with each input in the sequence having an associated timestamp. Preferably, the disease characterization model will provide an output for each input in the longitudinal data sequence, and the fusion module will provide a single aggregated output. To parse longitudinal data associated with T points in time, the disease characterization model (125) may output characterization vectors $v$ for each of the timepoints. That is, $v = [v_0, v_1, \ldots, v_{T-1}]$. In a further example, the longitudinal disease characterization is complemented with an encoded time stamp produced by a time stamp encoder (360) indicative of when the input data was acquired. The time stamp encoded vectors $t = [t_0, t_1, \ldots, t_{T-1}]$ can be aggregated with the characterization vectors $v$, for instance, through the characterization fusion circuit (320), resulting in an augmented characterization vector $v(t) = [(v_0, t_0), (v_1, t_1), \ldots, (v_{T-1}, t_{T-1})]$.

In some examples of this invention, the disease characterization model is an array of characterization models, each specialised to characterise a different category of features, or different characteristics of single feature category. A characteristic is here defined as a property with a numerical value, or a categorisation, that is associated with an instance of a feature. For the purposes of the disease characterisation model, it should be possible to determine or predict the characteristic from the input data (115). For example, a first disease characterization model may be dedicated the characterization of the lung lesion and its surrounding lung tissue, while a second disease characterisation model may be dedicated to characterizing the nodules of the lymphatic system.

### Outcome prediction

The functionality of outcome prediction consists in the estimation of a lung cancer outcome score (145) for the lung cancer, or potential lung cancer, described by the input data (115) for a given lung cancer intervention (130). The outcome prediction functionality is implemented by an outcome prediction model (135), which takes as input a mathematical description of the input data (115) as produced by the disease characterization model (125), and a lung cancer intervention (130), as encoded by an intervention encoder (132). Preferably, the outcome prediction circuit further comprises an intervention encoder, and the at least one intervention record and the details of the future intervention are provided to the intervention encoder, which encodes the intervention information before providing it to the outcome prediction model. In an embodiment, the intervention encoder is trained to map raw intervention information into a vector to enhance the semantics of the raw information.

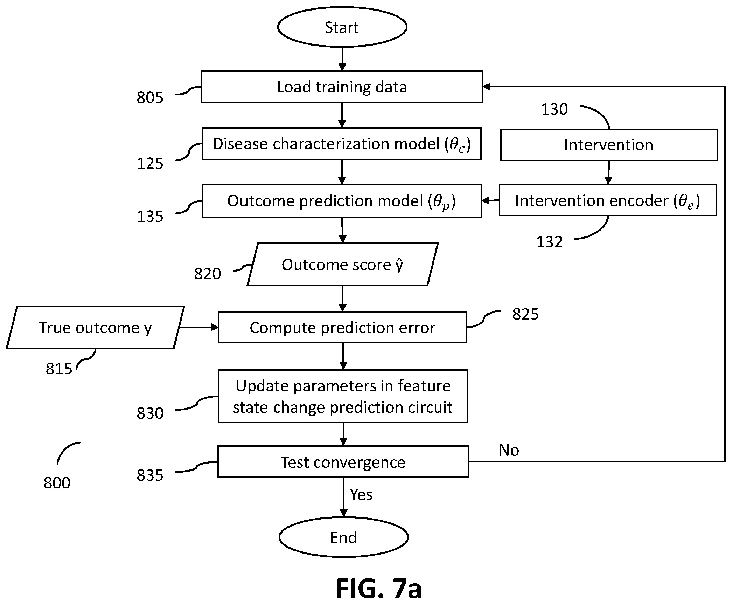

The outcome prediction model (135) is typically based on the statistical modelling of the input vectors $v$ produced by the disease characterization model (125). In an example of the invention, the outcome prediction model (135) is implemented with a machine learning model that maps disease characterization vectors $v$ to outcome scores (145). The mapping function of the outcome prediction model (135) is determined by the process of supervised training, in which it is trained from the error signal produced by predicting an outcome (145) on retrospectively collected training data, for instance as presented in FIG. 7a. That is, error signal is used to update the parameters of the outcome prediction model (135) according to an update algorithm such as backpropagation. This iterative process is repeated until convergence.

The outcome score (145) is typically indicative of the likelihood that the outcome being predicted would occur given an intervention (130). For example, it can consist of a continuous probability value given as a single number to the user, or stratified into levels of probability such as low, intermediate, and high probability. In other examples, the outcome score (145) will be in an arbitrary scale where higher numbers indicate higher likelihood of the outcome occurring.

In some examples of this invention, the outcome score (145) output by the outcome prediction model (135) is indicative of the likelihood that the lung cancer being analysed, and considered for a given intervention (130), will have a certain outcome within a pre-defined time $t_r$. For instance, when $t_r = 1$ year, and the outcome score is the probability of lung cancer recurrence, then the output of the CADx system (100) is interpreted as the probability that the lung cancer in the input data (115) would have recurred within a year of the given lung cancer intervention (130).

In some examples, the outcome score (145) is produced for a sequence of $R$ pre-defined times $T_r = [t_1, t_2, ... , t_R]$. This configuration allows to narrow down the time for the occurrence of the outcome predicted by the outcome score (145). For instance, if $T_r = [6months, 12months, 24 months]$, the outcome score (145) for a scenario of lung cancer recurrence could have low probability for the 6 months and 12 months buckets, but a high probability for the 24 months bucket, which is interpreted as an statistical estimate of recurrence at around 24 months after the intervention (130).

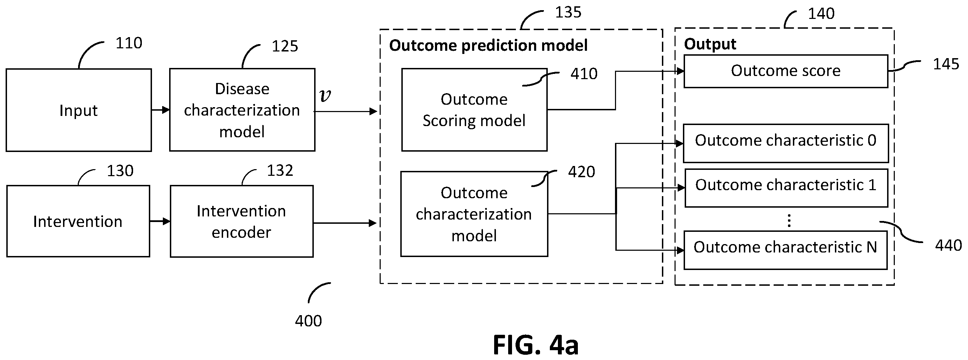

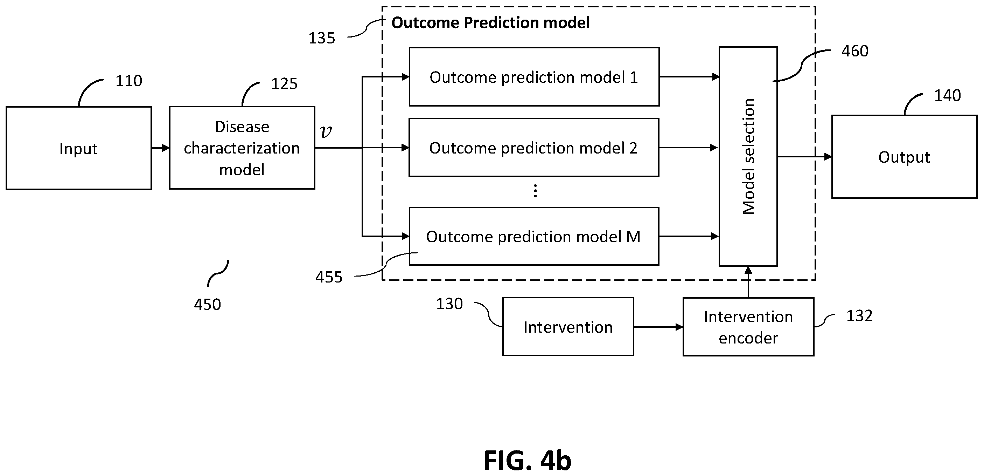

Some examples of the outcome prediction model (135) also output a characterization of the outcome (440) in addition to the outcome score (145), as depicted in FIG 4a (400). In some examples of this invention, this functionality is achieved by a configuration where the outcome prediction model (135) consists of an outcome scoring model (410), which computes the outcome score, and an outcome characterization model (420), which computes N different characterizations of the outcome (440). In another example of the invention (450) depicted in FIG 4b, the outcome characterization model (420) consists of N different models (455), each addressing a single characterization of the outcome, where a model selection circuit (460) selects which of the model outputs to pass on to the output circuit (140) for display to the user according to the CADx device configuration.. In a preferred configuration where the outcome score (145) of a lung cancer recurrence score, the outcome characterization consists of at least one of time-to-lung cancer recurrence, location of recurrence (e.g. local, regional, or distant), likelihood that the lesion is malignant, histological type cancer and specific mutations such as EGFR. Preferably, the outcome score is provided for a predefined time sequence between 1 month and 10 years.

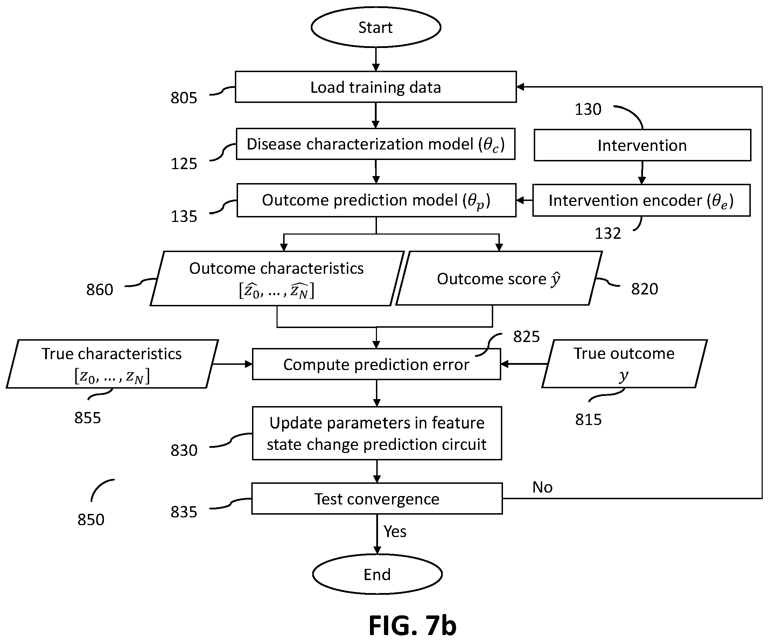

In a typical example of the outcome prediction model (135) that outputs disease characterization, the outcome characterization model (420) is built following a similar process to that of the outcome scoring model (410) as depicted in FIG 7b. That is, the parameters of the outcome characterization model (420) are determined by the process of supervised training using retrospectively collected training data, in which the prediction for each of the N characterizations of the disease are compared to the true characteristics (e.g., as determined by the clinical evaluation of the case) to produce an error signal. The error signal is then used to update the parameters of the outcome characterization model (420) according to an update algorithm such as backpropagation. This iterative process is repeated until convergence.

In an embodiment of the invention, the input data may be from either benign or malignant nodules as a final diagnosis for the lung lesion in the input data may not yet be available. In such a case, the outcome score (145) needs to be interpreted alongside the likelihood that the lesion is a lung cancer. For example, if the outcome score (145) indicates a high probability of lung cancer recurrence for an intervention (130) such as surgery, then a complimentary treatment such as neo-adjuvant therapy may be warranted. However, if the lesion is not yet diagnosed, and the likelihood of cancer is low, the outcome score may be considered non-actionable until the diagnosis is proven, or it could be considered a failure of the CADx device due to unexpected conditions. In this example, the outcome characterization model (420) is therefore a lung cancer diagnosis model, while the outcome scoring model (410) is a risk of recurrence model.

### Intervention encoding

The functionalities of disease characterization and outcome prediction of the outcome prediction circuit (120) allow for the computation of an outcome score (145) given an intervention (130) and, optionally, a history of interventions. However, a practical implementation of the outcome prediction circuit requires that the interventions (130), and intervention records (131) are appropriately encoded to be input into the outcome prediction model (135), for example, when the latter is implemented through a machine learning model.

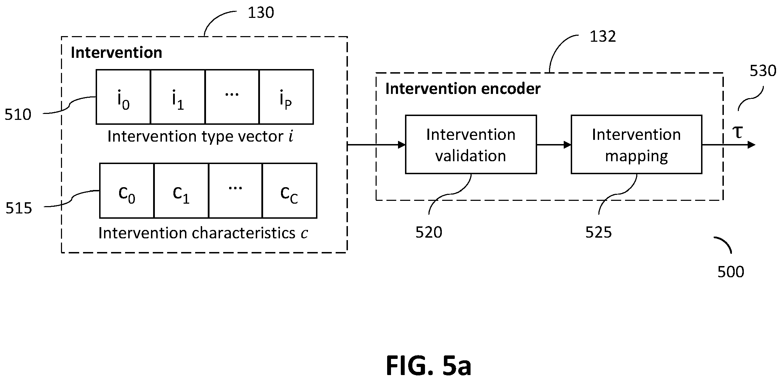

An example of the intervention encoder (132) takes the intervention (130) and categorizes it according to types of interventions (FIG. 5a) (500). For example, common intervention types for lung cancer include surgery, radiotherapy, chemotherapy, immunotherapy, ablation, laser therapy, cryotherapy, diathermy, and photodynamic therapy. The case where the clinical decision could be to not perform any intervention is also considered, and "nointervention" can be treated as a type of intervention. In a simple example of the intervention encoder (132), P types are considered, and encoded in a P-dimensional vector, referred to as the intervention type vector i (510), that indicates which of the types are being considered for a given lung cancer case. Here, each of the entries of the intervention type vector $i$ (510) (e.g.[$i_0$, $i_1$, ... , $i_{P-1}$]) corresponds to each of the P intervention types. In a basic example, the intervention vector i is a binary vector which has a value of 1 on the position associated with the intervention type being considered (130) while the remaining entries are set to the value of 0. In a further example, where more than one intervention type is considered simultaneously, all entries corresponding to the interventions considered are set to a non-zero values. In a further example, where the intervention vector i is a null vector, e.g. all set to 0, it is interpreted by subsequent circuits as no-intervention. In an embodiment, at least one of the historical intervention records and the future interventions are set as a null value, and are considered as no-interventions towards the calculations of the outcome score.

The intervention type vector i (510) is given as input to the intervention encoder (132) and typically checked for validity (520). That is, the validation checks that only combinations of the intervention type vector i (510) allowed by the manufacturer of the CADx system (100) are input. The restriction is typically the result of what combination of interventions are present in the data with which the statistical models powering the CADx system were developed. For example, the retrospective data available for the development of the outcome prediction model (135).

Once validated, the intervention type vector i (510) is input to the intervention mapping model (525) whose task is to map the vector i (510) into an encoded form $\tau$ (530) that can be used by the outcome prediction model (135). In a trivial example, where the intervention type vector i (510) is in a suitable form for the outcome prediction model (135) the intervention mapping (525) applies an identity transformation. In a preferred example, the intervention mapping (525) is built such that it optimizes the mapping form *i* to $\tau$ (530) to suit the outcome prediction task. For example, the intervention mapping (525) is built with a parametric model such as a machine learning model, and its parameters are learned from the error signal produced by predicting an outcome (145) on retrospectively collected training data. Such a mapping can perform a combination of operations on the intervention type vector i (510), including mapping it to a higher dimensional space where the semantic information in the vector i is enhanced.

In a further example of the intervention encoder (132), also depicted in FIG 5a, the input intervention (130) is augmented with further details of the intervention applied, as captured in an intervention characteristics vector c (515) which contains C characteristics [$c_0$, $c_1$, ... , $c_{C-1}$] of the intervention. For example, if the intervention type vector i (510) indicates a resection surgery, the intervention characteristics vector c (515) may indicate whether the surgery is a wedge resection, lobectomy, or lung resection. Likewise, if the intervention type vector i (510) indicates that radiotherapy will be performed, the intervention characteristics vector c (515) may indicate a type of radiotherapy such as external beam radiation, intensity modulated radiation therapy (IMRT), or stereotactic radiation techniques (SBRT/SABR). As a further example, when the intervention type vector i (510) indicates the application of chemotherapy, intervention characteristics vector c (515) may indicate a class and/or dose of the chemotherapy agent.

The intervention characteristics vector c (515) is typically also checked for validity (520) on the same grounds as the intervention type vector i (510), and to validate that the characterizations being input in c corresponds to the type of intervention indicated i. For instance, if the intervention type vector i (510) indicates that radiotherapy will be performed, the intervention characteristics vector c (515) indicates a radiation dose, then the validation (520) of the characteristic vector c (515) validates that the dose is within an accepted range.

In some examples of the intervention encoder, the vectors i (510) and c (515) are concatenated in the intervention mapping (525) model and mapped into a single encoded form $\tau$ (530) through a model built to optimize the mapping for the outcome prediction task. Preferably, the intervention record is provided to the intervention encoder (132) in the form of an intervention type vector (510) and an intervention characteristic vector (515). In an embodiment, the mapping of the intervention information into a vector is an identity mapping that does not change the intervention type vector or the intervention characteristic vector.

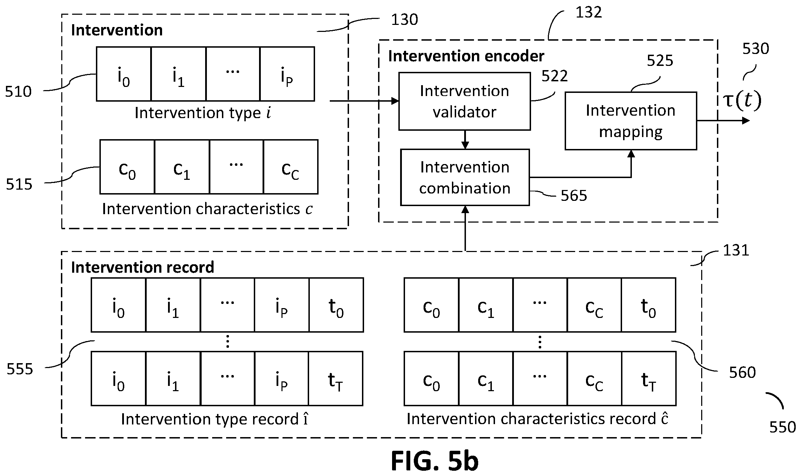

As depicted in FIG 5b, in an embodiment (550) of the intervention encoder the intervention information can include a record of previous interventions (131). This intervention record typically consists of sequences of the intervention type and characteristic vectors i (510) and c (515), which are augmented with time stamps that indicate the point in time at which each intervention was done, resulting in the corresponding time-augmented matrices $\hat{i}$ (555) and $\hat{c}$ (560). As such, the intervention record (131) is typically full accounting of all interventions that a lung cancer has received on a given patient, and when these occurred, which can be accounted for towards the prediction of a new outcome score (145) for a new intervention (130). In an example of this configuration, the validated intervention (130) is combined with the intervention record (131) in an intervention combination step (565), which may consist in the simple concatenation of vectors *i* (510) with $\hat{i}$ (555) and, if available, c (515) with $\hat{c}$ (560), and filling the time stamp information for the next intervention (130) with the present time stamp. Once combined, the full intervention array is processed by the intervention mapping (525) to produce a time-augmented intervention encoding $\tau(t)$ (530).

The various configurations of the intervention encoder (132) allow for the scenario where the intervention record (131) is empty, indicating no history of intervention, the intervention (130) is empty, indicating that a lack of intervention is being considered, or both. In some implementations of the intervention encoder, these scenarios are handled by setting the respective values of the intervention type vectors (e.g. *i* (510) and/or $\hat{i}$ (555)) to zero. In a different implementation, the no-intervention scenario is handled as a separate intervention type among the P possibilities.

### Joint training of the outcome prediction model based on machine learning

In a preferred embodiment of this invention, the disease characterization model (125), the outcome prediction model (135) and the intervention encoder (132) are based on neural networks with parameters $\theta_c$, $\theta_p$ and $\theta_e$ respectively, which are trained jointly and end-to-end. Preferably, one or more of the disease characterisation model and the outcome prediction model are trained using machine learning. A training process (800) of this general configuration of the invention is shown in FIG 7a. The training process requires a corpus of data, referred to as training data (805), consisting of a collection data sets (115). For instance, CT scans of patients showing their lungs and a suspicious lung lesion, alongside the real outcomes of such lesions, denoted as y (815). For example, whether the lung cancer recurred following a given intervention (130). The training process is an iterative one, where each iteration starts by loading samples of the training data (805) which are then processed by the disease characterisation model (125). Its output is then input to the outcome prediction model (135). The latter also takes as input information about the intervention (130) as provided by the intervention encoder (132). The outcome prediction model (135) issues an outcome score estimation (820) denoted as ŷ, which is aims to match the true outcome y (815). Therefore, the outcome estimation ŷ (820) and the true outcome y (815) are compared using a loss function which measures the prediction error (825). Such loss functions are typically classification loss functions such as cross-entropy loss, or regression loss functions such as L2 distance. The prediction error (825) is the used to update the parameters $\theta_c$, $\theta_p$ and $\theta_e$ such that the prediction error (825) is minimized. The update process is typically done using algorithms such as backpropagation in combination with a parameter update algorithm such as that of stochastic gradient descent. A training iteration finalizes with a test for convergence (835), which evaluates whether a stopping criterion has been reached. In some examples, the test for convergence consists of evaluating a metric such as prediction error on a subset of the training data, typically one that is not used to compute the prediction errors (825) that led to updating the various model parameters of the outcome prediction circuit (120). If this metric fulfils a criterion such as not having changed since the last iteration of the training process (800), then the process ends. Otherwise, another iteration is started.

This process is extended in FIG 7b to the configuration of the CADx system (100) where the outcome prediction model (135) also outputs N characterizations of the outcome. In this example configuration (850), the predicted outcome characteristics $[\widehat{z_0}, \ldots, \widehat{z_{N-1}}]$ (860) are compare with the true outcome characteristics $[z_0, \ldots, z_{N-1}]$ (855) in a similar way as comparing y (815) and ŷ (820). That is, computing a prediction error (825), for instance, using classification loss functions or regression loss functions according to the type of the characterization. For example, a classification loss function such as cross-entropy loss may be used to assess the prediction error of a characteristic consisting on whether a lung cancer recurrence is local, regional or distant. While a regression loss function such as L2 distance may be sued to assess the prediction error of a characteristic consisting of the time to an expected recurrence. Finally, the update of the various parameters of the machine learning models is done using a combination of the error signals provided by each of the prediction tasks; namely, the outcome prediction and the prediction of each of the outcome characteristics being considered.

## Embodiments of the outcome prediction circuit

The various configurations of the disease characterization model (125), outcome prediction model (135) and intervention (130), as encoded by the intervention encoding (132), can be combined to address specific clinical scenarios with the CADx system (100) of this invention. Examples of those are described next.

### Pre-diagnosis lung cancer recurrence prediction

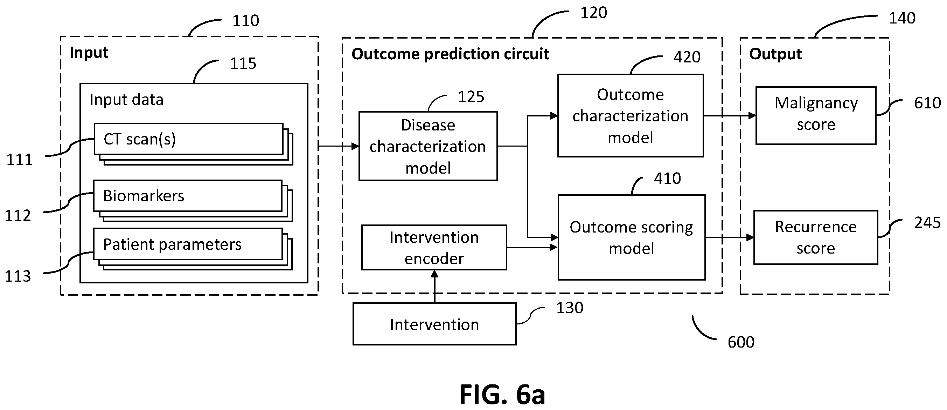

An embodiment of the CADx system (100) of this invention is used to predict an outcome of probability of lung cancer recurrence given an intervention of choice at the point when the lung lesion has been detected but not yet diagnosed. In this embodiment (600), presented in FIG 6a, the outcome scoring model (410) produces a recurrence score indicative of the likelihood that the lung lesion present in the input data (115) will recur following a given intervention (130), where no-intervention is also allowed. As the lung lesion has not been diagnosed, the outcome characterization model (420) produces a malignancy score (610) indicative of the likelihood that the lung lesion in the input data (115) corresponds to lung cancer. In this scenario, there is typically no intervention record (131) as the lesion has not even been diagnosed. Finally, the input data would typically consist of one or more CT scans (111), patient information (113) and potentially the result of some liquid biomarker (112). This embodiment is appropriate for use, for example, in multi-disciplinary tumour boards, typically containing radiologists, pulmonologists, medical oncologists, and surgeons, deciding on a treatment plan based on the risk of lung cancer recurrence. For instance, to decide to speed up a final diagnosis and begin treatment planning.

### Pre-operative lung cancer recurrence prediction

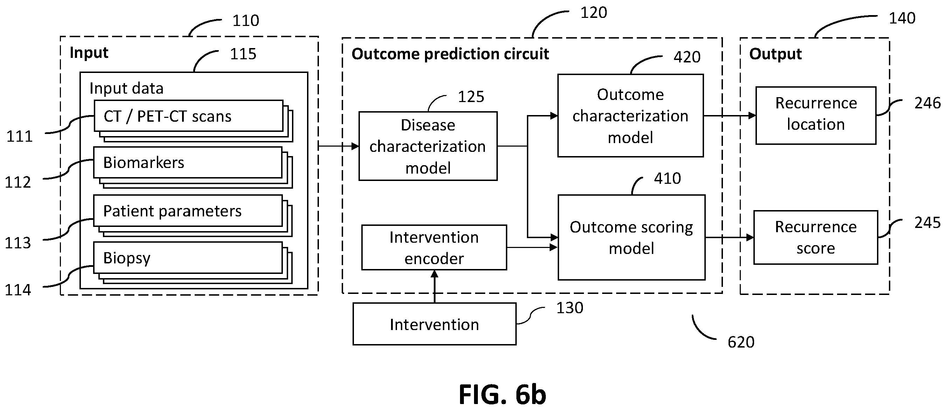

In this embodiment (620), illustrated in FIG. 6b, lung cancer has been either diagnosed or suspected with high confidence. Unlike the pre-diagnosis embodiment, the pre-operative embodiment typically has access to diagnostic images such as PET-CT (111), as well as the result of biopsies (114) performed to the lesion from liquid and/or tissue biopsy prior to surgical resection. This embodiment may be used when deciding on a treatment plan based on the risk of lung cancer recurrence. For instance, high-risk nodules can be pre-emptively treated with neo-adjuvant chemo- or radiotherapy.

### Post-operative lung cancer recurrence prediction

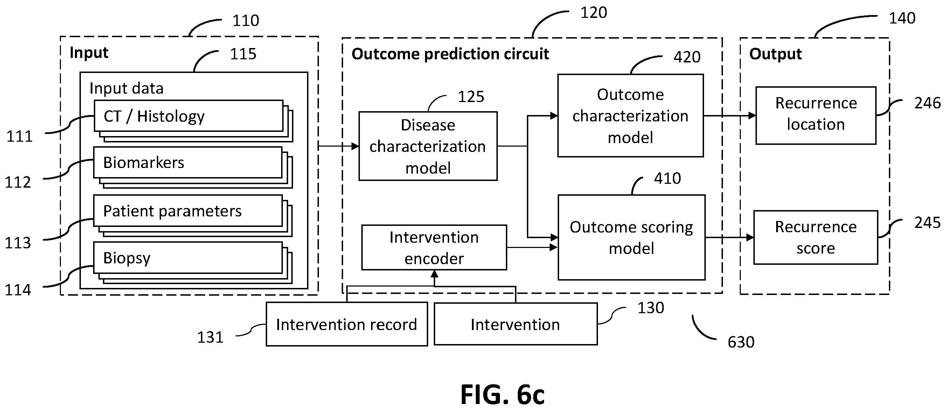

In this embodiment (630), illustrated in FIG. 6c, the lung cancer lesion has already been intervened at least with surgery, and hence there is an intervention record available (131). In an embodiment, the intervention record is a record of all interventions performed for the patient, along with the timestamp for each intervention; where the interventions in the intervention record and the future intervention comprise at least one of: surgery, radiotherapy, chemotherapy, immunotherapy, ablation, laser therapy, cryotherapy, diathermy, and photodynamic therapy, and no intervention. Typically, also histological images (111), and further diagnostic images performed after surgery would be available, aside from the pre-surgical images. A possible output in this embodiment consists of a recurrence score (245), for instance, as a probability from 0 to 1, and a prediction of recurrence location (246), for instance local vs distant. One potential used for this embodiment is by managing physicians in deciding on adjuvant treatment and follow-up plan. Following a treatment plan, the risk of recurrence can again be re-calculated based on the treatment provided, and further updated based on the results of follow-up CT images and/or liquid or tissue biopsy results.

### Pre-treatment response prediction

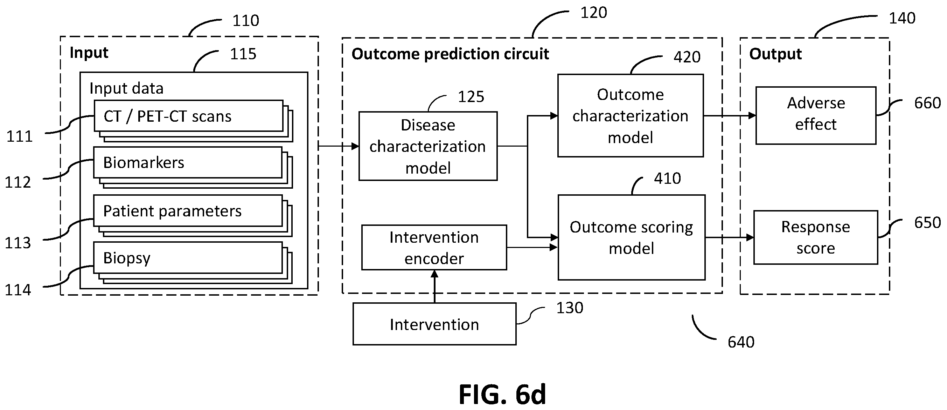

In this embodiment (640), illustrated in FIG. 6d, no treatment has been applied to a diagnosed lung cancer, and a range of drugs is considered, for example, for chemotherapy and immunotherapy. The main outcome to be scored (410) is the tumour response to the treatment in the form of a response score (650). For instance, as a probability among the response types of complete response, partial response, stability or disease progression. In addition to the response score (650), an outcome characterization to be predicted (420) consists of the adverse effects (660) of the intervention (130) drug. For instance, the probability and/or severity of an inflammatory response caused by the drug.

-ACADx device that computes a lung cancer outcome score (145) conditioned on a clinical intervention (130) is described above. In the CADx device the intervention information is made up of types of interventions (510) and characteristics of the intervention (515) which are input to the system as vectors. The intervention information may also account for a history of prior interventions (131) on the same lesion.

Preferably, the outcome score (145) from the CADx device is produced by the outcome prediction circuit (120) based on a machine learning model.

In an embodiment, the intervention information (130) is passed to the outcome prediction circuit (120) and processed by an intervention encoder (132) in the CADx device. Further preferably, the intervention encoder (132) is also a machine learning model that maps the intervention information (130) to a vector that enhances the semantic information of the intervention by being trained end-to-end alongside the outcome prediction model.

In an embodiment, the intervention encoder also accepts a record of time-stamped interventions (131) such that the outcome prediction circuit (120) can account for those in the outcome scoring. Preferably, the intervention encoder also accepts no-intervention as input. In an embodiment, the intervention encoder applies an identity mapping to the input intervention vector.

Preferably, the intervention encoder validates (520) the intervention information passed to it before applying the encoding.

In an embodiment, the outcome score (145) is accompanied by additional characterizations of the disease. For example, if the outcome score is for lung cancer recurrence, characterizations include whether the lung lesions is cancerous, it will recur locally or distantly, and an estimation of when it will recur.

Preferably, the CADx system outputs an estimate of when the outcome will occur based on the probability it will occur given for each of several buckets of time intervals.

In an embodiment, the input data is encoded by a disease characterization model (125). Preferably, the disease characterization model consists of an image characterization model (310) and a structured data characterization model (315), and their output is fused by a characterization fusion model (320).
where the disease characterization model is a longitudinal model that encodes sequences of images according to their timestamp.

The method and system as described are intended to aid clinicians on the very challenging task of predicting the outcome for a lung cancer patient following a clinical intervention. For instance, to determine if the lung cancer will recur after treatment and hence which patients should be given more or alternative interventions after the treatment, and which patients should be followed more closely.

The risk of recurrence of the lung cancer is typically mediated by the intervention that the clinicians perform. For example, an intervention of surgery alone vs an intervention of neo-adjuvant therapy plus surgery. Obtaining an accurate prediction of the risk of recurrence of the lung cancer given an intervention allows the clinician to make better informed decisions on the risk-to-benefit ratio of various interventions at their disposal. This is important because any lung cancer intervention has significant risk and side effects such as those of any invasive surgery, or the high toxicity cancer medication.

Similarly, the system and method as described can be used to determine the response of a lung cancer to a given intervention, for example, whether the disease will progress, stabilize, or respond partially or completely as a following a non-surgical intervention.

Therefore, by being able to predict the outcome of the lung cancer specifically for the intervention that is being considered for the patient, the clinicians can refine their assessment of the case and make better clinical decisions.

Finally, to extend the applicability of such a CADx system, it is important that it accounts for the clinical history of the patient's cancer; specifically, previous interventions that the patient has been subject to, as these would also be highly consequential for the prediction of future outcomes.

This invention enables such clinical decision support system by being able to condition the prediction of the cancer outcome, and any characteristic of such an outcome, on the intervention being considered and, if available, the patient's intervention record.

As discussed above, this invention aids clinicians on the very challenging task of predicting the outcome of a lung cancer intervention, such as which lung cancer patients will have a recurrence of lung cancer after surgical resection, or respond to a given drug-based treatment, and hence, which patients should be given more or alternative interventions, and which should be followed more closely.

Specific scenarios that can be considered using the method as described above include:

> * Aiding doctors to decide on a personalised treatment strategy for lung cancer patients. For instance, if a patient is predicted to have a high risk of recurrence after surgical resection of their lung cancer, a doctor may prescribe additional therapy e.g., neoadjuvant and/or adjuvant immunotherapy and/or chemotherapy.

> * Aiding doctors to decide on a personalised surveillance strategy for lung cancer patients after treatment. For instance, if a patient is predicted to have a high risk of recurrence after surgical resection of their lung cancer, a doctor may image the patient more frequently than they otherwise would.

> * Aiding doctors in deciding whether to perform certain tests on a patient, given the predicted characteristics of a disease. For instance, if a patient had a primary lung cancer tumour with a high risk of recurrence, a doctor may perform additional lymph node biopsies to ensure the lung cancer had not already spread to the lymph nodes.

> * Improving the accuracy of medical tissue- or blood-based tests by combining the output of the CADx device with the results of a test.

The system and method as described can be implemented in: Healthcare systems; Clinical trials; Clinical research organizations as well as other organizations that require the extraction of clinical data from hospital systems and networks.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least. including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for automated contouring of cone-beam CT images.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate. decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above-described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

**Claims**

1. A Computer Aided Diagnosis, CADx, system for predicting an outcome score for a patient, comprising:

   an input circuit configured to receive input data comprising at least one input medical image for a patient;

   an outcome prediction circuit operably coupled to the input circuit configured to receive input data from the input circuit for outcome prediction analysis;
   wherein the outcome prediction circuit is further configured to receive details of a suggested future intervention for the patient; and
   the input data to the outcome prediction circuit is analysed to generate the outcome score for the patient accounting for the details of the future intervention.

2. The computer Aided Diagnosis, CADx, System as claimed in claim 1, wherein the output prediction circuit comprises a disease characterisation model and an outcome prediction model; and the outcome prediction circuit is configured to receive input data from the input circuit for analysis by the disease

   characterisation model;
   wherein output from the disease characterisation model is provided to the outcome prediction model, to generate the outcome score for the patient.

3. The computer Aided Diagnosis, CADx, system as claimed in claim 2,
   wherein the outcome prediction model is further configured to receive at least one or more historical intervention records for the patient; wherein at least one of the historical intervention records and the future intervention are set as a null value, and are considered as no-interventions towards calculations of the outcome score.

4. The computer Aided Diagnosis, CADx, system as claimed in any preceding claim wherein the outcome score comprises at least one of score of disease recurrence and treatment response, and the outcome score further comprises one or more different characterizations of the outcome .

5. The computer Aided Diagnosis, CADx system, as claimed in claim 4 wherein the one or more different characterisations of the outcome comprise one or more of: a malignancy score, a disease recurrence score, a disease recurrence location, a disease recurrence time, and an adverse effects score.

6. The computer aided diagnosis CADx system as claimed in any preceding claim wherein the outcome score is provided for a predefined time sequence between 1 month and 10 years.

7. The computer Aided Diagnosis, CADx, system as claimed in any preceding claim wherein the input medical image is at least one of a CT scan, X-ray scan, ultrasound scan, MRI scan, SPECT scan, PET scan, in which all or part of a patients lungs are visible.

8. The computer Aided Diagnosis, CADx, system as claimed in any preceding claim wherein the outcome prediction circuit further comprises an intervention encoder, and the at least one intervention record and the details of the future intervention are provided to the intervention encoder, which encodes the intervention record and details of the future intervention before providing it to the outcome prediction model, wherein the intervention encoder is trained to map raw intervention information into a vector to enhance semantics of the raw intervention information.

9. The computer aided diagnosis CADx, system, according to claim 8 wherein the intervention record is provided to the intervention encoder in the form of an intervention type vector and an intervention characteristic vector.

10. The computer Aided Diagnosis, CADx, system as claimed in claim 2 or any claim dependent on claim 2, wherein the disease characterization model comprises at least one of: an image characterization model for analysing visual features of an input image; and a structured data characterization model to encode structured data into a mathematical representation.

11. The computer Aided Diagnosis, CADx, system as claimed in claim 10 wherein the disease characterization model further comprises a fusion model to aggregate an output from the image characterization model and the structured data characterization model.

12. The computer Aided Diagnosis, CADx, system as claimed in claim 2 or any claim dependent on claim 2 where one or more of the disease characterisation models and the outcome prediction model are trained using machine learning.

13. The computer Aided Diagnosis, CADx, system as claimed in any preceding claim wherein the input data is provided as a longitudinal data sequence with each input in the sequence having an associated timestamp, and a disease characterization model will provide an output for each input in the longitudinal data sequence, and the fusion module will provide a single aggregated output .

14. The computer Aided Diagnosis, CADx, system as claimed in claim 3 or any claim dependent on claim 3 wherein the intervention record is a record of all interventions performed for the patient, along with a timestamp for each intervention; where the interventions in the intervention record and the future intervention comprise at least one of: surgery, radiotherapy, chemotherapy, immunotherapy, ablation, laser therapy, cryotherapy, diathermy, and photodynamic therapy, and no intervention.

15. A computer implemented method for predicting an outcome score for a patient, using a computer Aided Diagnosis, CADx, system comprising the steps of:

receiving, at an input circuit, input data comprising at least one input medical image for a patient;
receiving at an outcome prediction circuit operably coupled to the input circuit, input data from the input circuit, wherein the input data is analysed by the outcome prediction circuit;
wherein the outcome prediction circuit is configured to receive details of a future intervention for the patient; and
analysing the inputs to the outcome prediction circuit to generate an outcome score for the patient accounting for the details of the future intervention.

**FIG. 1**

EP 4 679 452 A1

**FIG. 2**

Input

Input data

- Medical image(s) — 111
- Biomarkers — 112
- Patient parameters — 113
- Biopsy — 114

110
115

Disease characterization model

Image characterization — 310, $v_i$

Structured data characterization — 315, $v_s$

Characterization fusion — 320, $v$

125
300

**FIG. 3a**

**FIG. 3b**

**FIG. 4a**

EP 4 679 452 A1

**FIG. 4b**

**FIG. 5a**

**FIG. 5b**

**FIG. 6a**

EP 4 679 452 A1

**FIG. 6b**

**FIG. 6c**

**FIG. 6d**

FIG. 7a

**FIG. 7b**

# EUROPEAN SEARCH REPORT

**Application Number**

EP 25 18 3406

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GANDHI ZAINAB ET AL: "Artificial Intelligence and Lung Cancer: Impact on Improving Patient Outcomes", CANCERS, vol. 15, no. 21, 31 October 2023 (2023-10-31), page 5236, XP093237052, CH ISSN: 2072-6694, DOI: 10.3390/cancers15215236 * the whole document * | 1-15 | INV. G16H50/20 G16H50/30 |
| X | BERA KAUSTAV ET AL: "Predicting cancer outcomes with radiomics and artificial intelligence in radiology", NATURE REVIEWS CLINICAL ONCOLOGY, NATURE, NY, US, vol. 19, no. 2, 18 October 2021 (2021-10-18), pages 132-146, XP037675447, ISSN: 1759-4774, DOI: 10.1038/S41571-021-00560-7 [retrieved on 2021-10-18] * the whole document * | 1-15 | |
| A | EP 4 071 768 A1 (OPTELLUM LTD [GB]) 12 October 2022 (2022-10-12) * the whole document * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 31 October 2025 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 3406

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2025

| Patent document cited in search report | | Publication date | Patent family member(s) | | Publication date |
|---|---|---|---|---|---|
| EP 4071768 | A1 | 12-10-2022 | EP | 4071768 A1 | 12-10-2022 |
| | | | US | 2022327690 A1 | 13-10-2022 |

EPO FORM P0459

For more details about this annex : see Official Journal of the European Patent Office, No. 12/82

# EP 4 679 452 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MACMAHON, HEBER et al.** Guidelines for management of incidental pulmonary nodules detected on CT images: from the Fleischner Society 2017. *Radiology*, 2017, vol. 284 (1), 228-243
- **YANO T** ; **YOKOYAMA H** ; **INOUE T et al.** The first site of recurrence after complete resection in non-small-cell carcinoma of the lung. Comparison between pN0 disease and pN2 disease. *J Thorac Cardiovasc Surg.*, 1994, vol. 108 (4), 680-3
- **CANGEMI V** ; **VOLPINO P** ; **D'ANDREA N et al.** Local and/or distant recurrences in T1-2/N0-1 non-small cell lung cancer. *Eur J Cardiothorac Surg.*, 1995, vol. 9 (9), 473-8
- **SUGIMURA H et al.** Survival after recurrent NSCLC after complete pulmonary resection. *Annals of Thoracic Surgery*, 2007
- **HOFFMAN PC** ; **MAUER AM** ; **VOKES EE**. Lung cancer. *Lancet*, 2000

- **CARNIO S et al.** Prognostic and predictive biomarkers in early stage non small-cell lung cancer: tumor based approaches including gene signatures. *Translational Lung Cancer Research*, 2013
- **D.J. GIROUX** ; **R. RAMI-PORTA** ; **K. CHANSKY et al.** The IASLC Lung Cancer Staging Project: data elements for the prospective project. *J Thorac Oncol*, 2009, vol. 4, 679-683
- **GANDHI L** ; **RODRIGUEZ-ABREU D** ; **GADGEEL S** ; **ESTEBAN E** ; **FELIP E** ; **DE ANGELIS F et al.** Pembrolizumab plus Chemotherapy in Metastatic Non-Small-Cell Lung Cancer. *N Engl J Med*, 2018, vol. 378 (22), 2078-92
- **TSAY JJ** ; **WU BG** ; **SULAIMAN I** ; **GERSHNER K** ; **SCHLUGER R** ; **LI Y** ; **YIE TA** ; **MEYN P** ; **OLSEN E** ; **PEREZ L**. Lower Airway Dysbiosis Affects Lung Cancer Progression. *Cancer Discov.*, 11 November 2020, vol. 11 (2), 293-307